# EUROPÄISCHE PATENTSCHRIFT

(11) **EP 0 908 176 B1**
(45) Veröffentlichungstag und Bekanntmachung des Hinweises auf die Patenterteilung: **11.12.2002**
(21) Anmeldenummer: 98117718.1
(22) Anmeldetag: 18.09.1998
(51) Int. Cl.: A61K 9/08, A61K 31/445

(54) **Intravenöse Applikationsform von Thalidomid zur Therapie Immunologischer Erkrankungen**
Intravenous administration form of thalidomide for the therapy of immunologic diseases
Forme d'administration parentérale de thalidomide pour la thérapie des maladies immunologiques

(30) Priorität: 06.10.1997 DE 19743968
(43) Veröffentlichungstag der Anmeldung: 14.04.1999
(73) Patentinhaber: Grünenthal GmbH, 52078 Aachen (DE)
(72) Erfinder: Björkman, Sven, Dr., 223 61 Lund (SE); Höglund, Peter, Dr., 231 98 Klagstorp (SE); Eriksson, Tommy, Dr., 237 36 Bjärred (SE)

(56) Entgegenhaltungen:
- WO-A-92/14455
- WO-A-95/17154
- WO-A-97/37988
- DE-A- 4 211 812
- CHEMICAL ABSTRACTS, vol. 118, no. 2, 11. Januar 1993 Columbus, Ohio, US; abstract no. 11605, XP002089567 & M. KRENN ET AL.: "IMPROVEMENTS IN SOLUBILITY AND STABILITY OF THALIDOMIDE UPON COMPLEXATION WITH HYDROXYPROPYL-.BETA.-CYCLODEXTRIN" J. PHARM. SCI. , Bd. 81, Nr. 7, 1992, Seiten 685-689,

## Beschreibung

Die Erfindung betrifft eine parenterale Applikationsform von Thalidomid sowie ein Verfahren zu ihrer Herstellung.

Die überschießende Bildung des Zytokins TNF-α (Tumornekrosefaktor α) spielt eine zentrale Rolle in der Pathogenese des Graft-versus-Host-Syndroms. aphthöser Stomatitis, Erythema nodosum leprosum, Morbus Boeck, Morbus Crohn, rheumatoider Arthritis und einer Reihe weiterer Erkrankungen, die mit entzündlichen Erscheinungen einhergehen. Ein Ansatz für die Therapie dieser Erkrankungen besteht in der gezielten Unterdrückung der TNF-α-Freisetzung durch Gabe immunmodulierender Wirkstoffe, wie zum Beispiel Dexamethason oder Thalidomid. Während Kortikoide wie Dexamethason in injizierbaren Formen vorliegen, ist dies für Thalidomid bisher nicht der Fall

Thalidomid hat sich in der Behandlung schwerer aphthöser Stomatitis im Vergleich zu den klassischen Immunsuppressiva als überlegen herausgestellt. Weitere Beispiele von Erkrankungen, in denen Thalidomid eine gute Wirksamkeit zeigte, ohne zu einer generellen Immunsuppression zu führen, sind kutaner Lupus erythematodes, Pyoderma gangränosum und orogenitale Ulcera bei Morbus Behcet sowie histologisch von aphthösen Ulzera nicht abweichende Ulzerationen bei HIV-Infizierten, in denen- anders als bei der Mehrzahl der HIV-assoziierten mukokutanen Läsionen - keine mikrobiellen Erreger nachgewiesen werden können. Diese zum Teil auch die Grösse von Major-Aphthen annehmenden Läsionen können im Unterschied zur Stomatitis aphthosa im gesamten Verdauungstrakt auftreten und bei Lokalisation im Rachenraum oder der Speiseröhre durch ihre Schmerz verursachende Wirkung die Nahrungsaufnahme, aber auch die orale Einnahme erschweren. Als pathogenetische Faktoren gelten endogene Mediatoren mit Wirkungen auf das Endothel und zirkulierende Leukozyten. Unter dem Einfluß von lokal gebildetem TNF-α und anderer Zytokine nimmt fokal die Adhäsivität des Endothels gegenüber Leukozyten zu, was maßgeblich zur Ausbildung venöser Vaskulitiden beiträgt. Substanzen, die wie Thalidomid diese Veränderung des Endothels unterdrücken, ohne zugleich die spezifische zelluläre Immunabwehr zu blockieren, können einen bedeutenden Fortschritt für die Therapie darstellen

Für schwere Fälle pharyngealer oder ösophagealer Ulzera, bei denen die orale Einnahme erschwert, wenn nicht sogar unmöglich sein kann, und für Fälle HIVassoziierter Pathologie, bei denen schwere Durchfallsymptomatik die orale Einnahme unberechenbar macht, bietet sich die parenterale Wirkstoffgabe an Die geringe Wasserlöslichkeit von Thalidomid (0,012 mg/ml. Arch Pharm 321, 371 (1988)) steht jedoch der parenteralen Applikation dieses Wirkstoffes entgegen. Es fehlte deshalb nicht an Versuchen, wasserlösliche Applikationsformen zu entwickeln.

Aus DE 42 11 812 sind wasserlösliche Thalidomidderivate bekannt, die im Vergleich zu Thalidomid eine deutlich höhere Wasserlöslichkeit aufweisen und zur parenteralen Applikation geeignet sind

Weiterhin wurden für die parenterale Applikation Thalidomid-Prodrugs vorgeschlagen, die im physiologischen pH-Wertbereich wasserlöslich applizierbar und toxikologisch unbedenklich sind (DE 196 13 976). Nachteilig ist, daß beide Arten der o.g. Verbindungen in ihrer Herstellung aufwendiger sind als die Herstellung von Thalidomid.

Aus Krenn et. al. (J. Pharm. Sci. 81, 685-689 (1992)) ist eine wässrige Thalidomidlösung gemischt mit hydroxypropyl-beta-cyclodextrin bekannt.

Die der Erfindung zugrundeliegende Aufgabe bestand in der Entwicklung einer wasserlöslichen Applikationsform von Thalidomid. Darüber hinaus soll die zu entwickelnde Applikationsform in wäßrig gelöster Form stabil sein und durch unphysiologische physikalisch-chemische Eigenschaften sollen keine toxikologischen Wirkungen hervorgerufen werden.

Es wurde gefunden, daß die an die zu entwickelnde Applikationsform gestellten Anforderungen unter bestimmten Bedingungen durch Verwendung reiner Enantiomere des Thalidomids erfüllt werden. Reine Enantiomere im Sinne dieser Erfindung enthalten weniger als 1% ihres optischen Antipoden.

Die Enantiomeren des Thalidomid weisen eine bis zum Faktor von 6 höhere Wasserlöslichkeit auf als das Racemat. Die Herstellung wäßriger Lösungen ist aber durch die Neigung Thalidomids zur spontanen Hydrolyse nicht praktikabel. Liegen die pH-Werte von wäßrigen Lösungen jedoch im Bereich von pH kleiner/gleich 5,5 bleibt die Hydrolyse aus.

Nach dem heutigen Wissensstand kann die Wirkungsweise Thalidomids bei immunologischen Erkrankungen nicht einem bestimmten Isomer zugeordnet werden. Reine Enantiomere des Thalidomids re-racemisieren in vitro und in vivo. Daher entsteht bereits unmittelbar nach parenteraler Gabe eines der Thalidomidisomeren in vivo auch der Antipode, nach etwa 4 Stunden hat sich ein Gleichgewicht eingestellt.

Gegenstand der Erfindung ist dementsprechend eine zur parenteralen Applikation geeignete Lösung eines der beiden Thalidomidenantiomeren 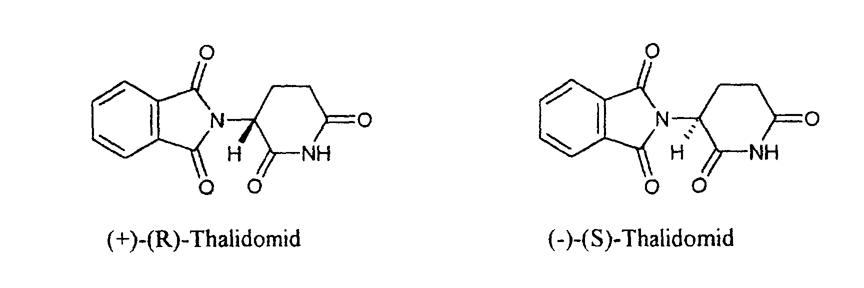 wobei diese Lösung eine wäßrige mit einem pH-Wert kleiner oder gleich 5,5 ist und als Bestandteil Glukose enthält. Erfindungsgemäß ist eines der beiden Thalidomidenantiomeren in isotonischer Glukoselösung gelöst.

Die Definition der Erfindung umfaßt sowohl Lösungen des (+)-(R)-Thalidomids als auch Lösungen des (-)-(S)-Thalidomids, die einzeln oder alternierend für die parenterale, insbesondere für die intravenöse. Applikation genutzt werden können.

Als injizierbare Applikationsformen von Thalidomid eignen sich solche, die einen Wirkstoffgehalt von mindestens 0,2 mg/ml aufweisen.

Weiterer Erfindungsgegenstand ist ein Verfahren zur Herstellung der wäßrigen Thalidomidlösung. Dabei wird erfindungsgemäß (+)-(R)-Thalidomid oder (-)-(S)-Thalidomid in reiner Form in eine isotonische Glukoselösung mit einem pH-Wert von 4 bis 5 gegeben und diese Mischung bis zur vollständigen Auflösung des jeweiligen Thalidomidenantiomeren geschüttelt, anschließend mit Ultraschall behandelt und unter aseptischen Bedingungen filtriert.

Die erfindungsgemäße Applikationsform ist sowohl bei schneller als auch bei langsamer Infusion (10 ml/min) toxikologisch unbedenklich

Erfindungsgemäße Arzneimittel enthalten neben einem der Thalidomidenantiomeren Glukose. Gegebenenfalls können der Thalidomidlösung weitere Hilfsstoffe zugesetzt werden. Die Auswahl weiterer Hilfsstoffe sowie die einzusetzenden Mengen hängen davon ab, wie das Arzneimittel genau appliziert werden soll

Die an den Patienten zu verabreichende Wirkstoffmenge, die vom Gewicht des Patienten, von der parenteralen Applikationsart, der Indikation und dem Schweregrad der Erkrankung abhängt, liegt üblicherweise zwischen 0,1 und 1 mg/kg.

### Beispiele

### Beispiel 1

Zur Herstellung von Infusionslösung in einer Konzentration von 200 µg/ml wurden 70 mg (+)-(R)-Thalidomid in 350 ml einer 5%igen Glukoselösung für Infusionen (pH 4 bis 5) in einer gläsernen Infusionsflasche gegeben. Die Mischung wurde gründlich geschüttelt und 15 Minuten mit Ultraschall behandelt. Da die erreichte gelöste Thalidomidkonzentration von der Intensität des Schüttelns und der Ultraschallbehandlung abhängt, wurden beide Schritte bis zur vollständigen Lösung wiederholt. Die Wassertemperatur im Ultraschallbad erreichte maximal 33° C. Die Lösung wurde unter aseptischen Bedingungen durch einen Millex GS Sterilfilter mit 0,22 µm Porengrösse (Millipore S.A., Molsheim, Frankreich) in eine sterile gläserne Infusionsflasche filtriert. Die Lösung wurde bei Raumtemperatur aufbewahrt.

Der pH-Wert der fertigen Lösung betrug 5,5.

Die Zeitdauer der Ultraschallbehandlung kann reduziert werden, indem eine Lösung des reinen Enantiomeren in Ethanol eingesetzt wird, die eine um den Faktor 5 bis 10 höhere Ausgangskonzentration hat.

### Beispiel 2

Zur Herstellung von Infusionslösung in einer Konzentration von 200 µg/ml wurden 70 mg (-)-(S)-Thalidomid in 350 ml Glukoselösung für Infusionen (pH 4 bis 5) in einer gläsernen Infusionsflasche gegeben. Es wurde wie in Beispiel 1 verfahren.

Der pH-Wert der fertigen Lösung betrug 5,5.

### Stabilitätsprüfung

An 10 aufeinanderfolgenden Tagen wurden den Lösungen täglich Teilmengen zur Analyse entnommen

Nach den 10 Tagen lagen die jeweiligen Thalidomidenantiomeren noch völlig intakt vor, ohne daß Hydrolyse eingetreten war.
Die Thalidomidenantiomeren enthielten nach diesem Zeitraum weniger als 1 % ihrer optischen Antipoden.

## Patentansprüche

1. Wäßrige Thalidomidlösung mit einem pH-Wert von kleiner oder gleich 5,5, bei der eines der beiden Thalidomidenantiomeren (+)-(R)-Thalidomid oder (-)-(S)-Thalidomid in reiner Form in isotonischer Glukoselösung gelöst ist.

2. Wäßrige Thalidomidlösung gemäß Anspruch 1, **dadurch gekennzeichnet, daß** sie einen Wirkstoffgehalt von mindestens 0,2 mg/ml aufweist.

3. Verfahren zur Herstellung der wäßrigen Thalidomidlösung gemäß Anspruch 1 oder 2, **dadurch gekennzeichnet, daß** (+)-(R)-Thalidomid oder (-)-(S)-Thalidomid in reiner Form in eine isotonische Glukoselösung mit einem pH-Wert von 4 bis 5 gegeben und diese Mischung bis zur vollständigen Auflösung des jeweiligen Thalidomidenantiomeren geschüttelt, anschließend mit Ultraschall behandelt und unter aseptischen Bedingungen filtriert wird.

## Claims

1. Aqueous thalidomide solution having a pH value of less than or equal to 5.5, in which one of the two thalidomide enantiomers (+)-(R)-thalidomide or (-)-(S)-thalidomide in pure form is dissolved in isotonic glucose solution.

2. Aqueous thalidomide solution according to claim 1, **characterised in that** it has an active ingredient content of at least 0.2 mg/ml.

3. Process for the preparation of the aqueous thalidomide solution according to claim 1 or 2, **characterised in that** (+)-(R)-thalidomide or (-)-(S)-thalidomide in pure form is added to an isotonic glucose solution having a pH value of from 4 to 5 and the mixture is shaken until the thalidomide enantiomer in question has dissolved completely, and the solution is then treated with ultrasound and filtered under aseptic conditions.

## Revendications

1. Solution aqueuse de thalidomide ayant une valeur de pH inférieure ou égale à 5,5, dans laquelle l'un des deux énantiomères du thalidomide, (+)-(R)-thalidomide ou (-)-(S)-thalidomide, est dissous sous la forme pure dans une solution isotonique de glucose.

2. Solution aqueuse de thalidomide suivant la revendication 1, **caractérisée en ce qu'**elle présente une teneur en substance active d'au moins 0,2 mg/ml.

3. Procédé de préparation de la solution aqueuse de thalidomide suivant la revendication 1 ou 2, **caractérisé en ce qu'**on ajoute du (+)-(R)-thalidomide ou du (-)-(S)-thalidomide sous la forme pure à une solution isotonique de glucose à une valeur de pH de 4 à 5 et on agite ce mélange par secousses jusqu'à ce que l'énantiomère de thalidomide concerné soit complètement dissous, puis on traite la solution aux ultrasons et on la filtre dans des conditions aseptiques.
